# EUROPEAN PATENT SPECIFICATION

(11) **EP 2 774 476 B1**
(45) Date of publication and mention of the grant of the patent: **12.10.2016**
(21) Application number: 12845098.8
(22) Date of filing: 30.10.2012
(51) Int. Cl.: A01G 7/00, A01N 37/46, A01N 63/00, A01P 21/00, G01N 33/92

(54) **MANAGEMENT METHOD AND MANAGEMENT SYSTEM FOR BIOMASS AT PLANT HARVEST**
VERWALTUNGSVERFAHREN UND VERWALTUNGSSYSTEM FÜR BIOMASSE BEI EINER PFLANZENERNTE
PROCÉDÉ DE GESTION ET SYSTÈME DE GESTION DE LA BIOMASSE LORS DE LA RÉCOLTE DE PLANTES

(30) Priority: 02.11.2011 JP 2011241633
(43) Date of publication of application: 10.09.2014
(73) Proprietor: Japan Science and Technology Agency, Kawaguchi-shi Saitama 332-0012 (JP)
(72) Inventor: OGAWA, Kenichi, Kyoto-shi Kyoto 600-8853 (JP)
(74) Representative: Cabinet Plasseraud
(86) International application number: PCT/JP2012/078073
(87) International publication number: WO 2013/065697

(56) References cited:
- JP-A- 2000 300 077
- JP-A- 2010 166 851
- JP-B2- 4 094 971
- JP-B2- 4 095 112
- JP-B2- 4 621 891

## Description

### Technical Field

The present invention relates to a method and system for managing plant biomass at harvest (biomass at plant harvest) on the basis of the percentage of linolenic acid in a plant with respect to the total amount of fatty acids in the plant.

### Background Art

Plants have been deeply involved in humans as food, as well as, for example, ornamentals, fuels, and industrial materials such as paper and chemicals etc. Controlling and determining the germination, maturation and flowering times of plants are very important in estimating the harvest of ornamentals and food plants such as vegetables. Furthermore, in order to control yields of fruits etc., it is essential to estimate a year in which trees bear a lot of fruits (a year of good harvest).

It has been generally known, as a knowledge from experience, that the yields of crops and fruits depend to a great degree on meteorological factors etc. There has been known a relationship between meteorological factors and the amounts of open flowers and fruits, and, in practice, various estimations have been performed on growing conditions of plants on the basis of that relationship. However, these estimations themselves are not accurate enough, and also cannot sufficiently adapt to recent climate changes such as increasingly abnormal weather. Therefore, it has been becoming more difficult to estimate growing conditions of plants.

Under such circumstances, there have been strong demands to (i) identify a factor that more directly reflects plant biomass (e.g., the amount of fruits on a plant) at harvest than methods utilizing meteorological factors etc. and (ii) develop a method which utilizes the factor.

The present inventors prepared variants of Arabidopsis thaliana, qualitatively detected the amount of active oxygen by the method of Ogawa et al. (Non-patent Literature 1), and selected variants that contained larger amounts of active oxygen than the wild-type. These variants bloomed earlier than the wild-type in long-day, low-light conditions. The inventors have found that plants tend to bloom early when they contain excess amounts of active oxygen. Furthermore, an analysis of the variants showed that a gene responsible for the variants is linolenic acid synthetase (Patent Literature 1). Linolenic acid is a kind of fatty acids contained in plants.

Furthermore, the present inventors have revealed the existence of a newly-found mechanism to control the flowering of plants. That is, the inventors have revealed the existence of a fatty acid composition of biomembranes as a new trigger for the expression of a flowering pathway which has been considered to take place under control of a flowering control gene. The fatty acid composition in biomembranes is, specifically, the percentage of linolenic acid with respect to total fatty acids. On the basis of this finding, the inventors have made it possible, by measuring the amount of linolenic acid contained in leaves at a time when flower buds of a plant are about to form, to accurately estimate the flowering time of the plant prior to the formation of flower buds (Patent Literature 2).

### Citation List

### Patent Literatures

Patent Literature 1
   Japan Patent No. 4094971 B/Japanese Patent Application Publication, Tokukai, No. 2004-264245 A (Publication Date: September 24, 2004)
Patent Literature 2
   Japan Patent No. 4095112B/Japanese Patent Application Publication, Tokukai, No. 2008-70384 A (Publication Date: March 27, 2008)

### Non-patent Literature

Non-patent Literature 1
Ogawa et al. 2001 Plant and Cell Physiology 42: 286-291 (Published in March 2001)

### Summary of Invention

### Technical Problem

In the field of agriculture, there has been a strong demand for a technique that allows for early knowing of productivity. Early knowing of productivity would make it possible to avoid overproduction and underproduction etc. and thus achieve a further improvement in profitability. However, neither of the methods disclosed in Patent Literatures 1 and 2 allows the direct knowing of plants' productivity, although they can be used to know growing conditions of plants and estimate the flowering time of the plants.

The present invention has been made in view of these problems, and an object of the present invention is to provide a method and system for knowing in advance plant biomass at harvest and managing the plant biomass.

### Solution to Problem

The inventors of the present invention have diligently conducted a study on a biological indicator that can be used to know in advance the plant biomass at harvest. As a result, the inventors have found that the plant biomass at harvest can be known accurately in a relatively early stage on the basis of the percentage of linolenic acid in a plant with respect to the total amount of fatty acids in the plant and that this percentage of linolenic acid is suitable as the indicator.

That is, in order to attain the above object, a method in accordance with the present invention is a method for managing plant biomass at harvest, including the steps of: a) measuring an amount of fatty acids contained in a plant(s); b) obtaining a percentage of linolenic acid with respect to a total amount of fatty acids that is found from the measurement; and c) estimating the plant biomass at harvest on the basis of the percentage of linolenic acid thus obtained.
A system in accordance with the present invention is a system for managing plant biomass at harvest, including: measuring means for measuring an amount of fatty acids contained in a plant(s); obtaining means for obtaining a percentage of linolenic acid with respect to a total amount of fatty acids that is found from the measurement; and estimating means for estimating the plant biomass at harvest on the basis of the percentage of linolenic acid thus obtained.

### Advantageous Effects of Invention

The present invention provides a method and system for knowing in advance plant biomass at harvest and managing the biomass at harvest.

### Brief Description of Drawings

Fig. 1 is a block diagram schematically illustrating a management system in accordance with one embodiment of the preset invention.
Fig. 2 is a graph showing an absorption spectrum of water in a near-infrared region and a waveform obtained by second-order differentiation of the spectrum.
Fig. 3 is a graph providing a comparison of peak area derived from linolenic acid and peak area derived from water.
Fig. 4 is a graph showing absorption spectra of a leaf of an orange tree in a near-infrared region.
Fig. 5 is a graph showing a waveform obtained by second-order differentiation of an absorption spectrum of a leaf of an orange tree in a near-infrared region.
Fig. 6 is a graph showing absorption peaks of fatty acids in a leaf of an orange tree in a near-infrared region.
Fig. 7 is a graph showing the results obtained by gas chromatographically analyzing the amounts of fatty acids in Arabidopsis thaliana individuals containing different amounts of linolenic acid.
Fig. 8 is a graph showing absorption peaks derived from fatty acids in three kinds of coniferous trees.
Fig. 9 is a graph showing how the application of glutathione to Arabidopsis thaliana is effective.
Fig. 10 shows the relationship between the percentage of linolenic acid in orange and the number of flowers on orange trees.
Fig. 11 shows the relationship between the percentage of linolenic acid in Dahurian larch and the amount of cones on Dahurian larch trees.
Fig. 12 shows the relationship between the percentage of linolenic acid in Dahurian larch and the male flowers/female flowers ratio of Dahurian larch.
Fig. 13 is a graph showing waveforms obtained by second-order differentiation of absorption spectra of Arabidopsis thaliana leaves containing different amounts of linolenic acid, in a near-infrared region.

### Description of Embodiments

### [1. Method for managing plant biomass at harvest]

### (Overview of the method)

A method for managing plant biomass at harvest in accordance with the present invention includes the steps of: measuring an amount of fatty acids contained in a plant(s); obtaining a percentage of linolenic acid with respect to a total amount of fatty acids that is found from the measurement; and estimating plant biomass at harvest from the percentage of linolenic acid thus obtained.

The method may further include, if needed, a step of judging, after the estimating step. The step of judging judges, by comparing the biomass at harvest estimated in the estimating step with target biomass at harvest (target value of the biomass at harvest), whether or not it is necessary to regulate the plant biomass at harvest.

The method may further include, if needed, a step of regulating the biomass. On the basis of the biomass at harvest estimated in the estimating step, the step of regulating performs a process to cause the plant biomass at harvest to be closer to the target value. One example of the regulating step is changing the glutathione concentration in a plant, in particular, changing the daily pattern of glutathione concentration, while keeping the plant growing even after the estimating step and the judging step until when the plant is made use of. The plant biomass at harvest is regulated by changing the glutathione concentration of the plant. Another example of the regulating step is to stop growing the plant after the judging step to thereby prevent overproduction of the plant. The following description will discuss in more details each step and the like in accordance with the present invention.

### (Measuring step)

The measuring step is a step of destructively or nondestructively measuring the amount of fatty acids contained in a plant. The amount of each fatty acid contained in a plant is measured, and the total amount of fatty acids contained in the plant is found from the amounts of these fatty acids thus measured.

The destructive measurement is a method of (i) taking a sample (part of a plant body) from a plant that is to be measured and (ii) measuring, preferably directly, the amount of fatty acids contained in the sample. The amount of fatty acids can be measured by, for example, physicochemical determination such as gas chromatography, thin-layer chromatography or acid-base titration. Of these, gas chromatography is preferable (also refer to the descriptions of Patent Literatures 1 and 2). The destructive measurement of the amount of fatty acids is advantageous in that it is possible to perform a direct analysis.

The nondestructive measurement is a method of measuring the amount of fatty acids contained in a plant to be measured, without destroying the plant (i.e., taking a sample is not necessary). The amount of fatty acids can be measured by, for example, near-infrared spectroscopy. As compared to the destructive measurement, the nondestructive measurement requires relatively less expertise and special instruments in preparing and analyzing a sample, and gives the results of the analysis in a relatively short period of time. Furthermore, in the nondestructive measurement, there is no possibility that the sample that is to be analyzed deteriorates due to stress caused by damage when the sample is taken out (cut out) from the plant. The nondestructive measurement is advantageous also in that, since the nondestructive measurement does not necessitate sampling, it is possible to perform fixed-point measurements on an individual plant over a period of time.

The following description specifically discusses one example of near-infrared spectroscopy, which is a kind of nondestructive method. The nondestructive measurement using near-infrared spectroscopy, which is employed in the present invention, is to nondestructively measure the amount of fatty acids contained in a plant. This method includes a step of spectroscopically analyzing at least a part of near-infrared light (which belongs to a wavelength range of not less than 1.250 µm but not more than 2.600 µm) contained in light reflected from a plant. This method can be performed also by utilizing a phenomenon in which natural light is reflected or absorbed by the surface of a plant, and therefore is advantageous in that it can be performed at a height (e.g., from an airplane or artificial satellite). Alternatively, it is possible to employ a method utilizing a phenomenon in which certain-wavelength light is absorbed also when light irradiated to a plant passes through it, i.e., a method including a step of spectroscopically analyzing at least a part of near-infrared light (which belongs to the wavelength range of not less than 1.250 µm but not more than 2.600 µm) contained in light that has passed through a plant. It should be noted that, although the following description is mainly based on a case where light reflected from a plant is used, usable peak wavelengths etc. are the same in the case where light reflected from a plant is used and in the case where light that has passed through a plant is used.

The inventors of the present invention have performed a detailed spectroscopic analysis of the light reflected from a plant in terms of an absorption spectrum in the near-infrared wavelength range of not less than 1.250 µm but not more than 2.600 µm. As a result, the inventors for the first time found that, in this range, there is a peak(s) characteristic of fatty acids and substantially unaffected by absorption by water. On the basis of this finding, the inventors showed that it would be possible to measure, by analyzing these peaks, the amount of fatty acids contained in a plant and the percentage of the amount of linolenic acid with respect to the total amount of fatty acids, without destroying the plant. Note, here, that the absorption spectrum represents a relationship between the wavelength of light irradiated to a plant (light wavelength) and absorbance. It should be noted that a peak(s) characteristic of fatty acids which peak(s) is affected by absorption by water can also be used to determine (i) the amount of fatty acids contained in a plant and (ii) the percentage of the amount of linolenic acid with respect to the total amount of fatty acids, without destroying the plant. However, the peaks affected by absorption by water (e.g., water vapor in the air) are used preferably in an embodiment which analyzes light that has passed through a plant at a close distance from the plant.

The "spectroscopic analysis" means obtaining an absorption spectrum by performing a spectral analysis on at least a part of near-infrared light (which belongs to the wavelength range of not less than 1.250 µm but not more than 2.600 µm) contained in the light reflected from a plant in order to analyze the light absorption by the plant. That is, the "spectroscopic analysis" means an analysis by near-infrared spectroscopy. As mentioned above, an absorption spectrum may be obtained by performing a spectral analysis on at least a part of near-infrared light (which belongs to the above wavelength range) contained in light that has passed through a plant.

Light reflected from a plant or light that has passed through a plant may be obtained from natural light or artificial light that contains near-infrared light belonging to the wavelength range of not less than 1.250 µm but not more than 2.600 µm. The near-infrared light that belongs to the wavelength range of not less than 1.250 µm but not more than 2.600 µm, which is to be analyzed, passes through a living body (plant body) well. Therefore, it is possible to obtain useful information even from a relatively thick plant body. It should be noted that, from the viewpoint of improving accuracy of the measurement or enabling the measurement under any conditions (e.g., dark condition), artificial light is preferable.

The spectroscopically obtained absorption spectrum may be differentiated twice (second-order differentiation) so that the spectrum more clearly indicates the fatty acids contained in the sample. However, depending on the purposes, the spectroscopically obtained absorption spectrum may be directly subjected to a comparative analysis. The waveform obtained by second-order differentiation and the positions of peaks obtained by second-order differentiation may vary depending on the amounts of substances contained in a target to be measured, composition of the target, and conditions of the differentiation etc. Suitable conditions can be selected as appropriate. How to perform second-order differentiation is not particularly limited, and the Savitzky-Golay method can be employed, for example.

In order to substantially eliminate the influence of water contained in a plant, it is preferable to perform the measurement based on at least a part of an absorption spectrum that is obtained by irradiating a plant with light which belongs to Wavelength Range A. Wavelength Range A is at least one selected from the group consisting of: the wavelength range of not less than 1.250 µm but not more than 1.340 µm; the wavelength range of not less than 1.355 µm but not more than 1.390 µm; the wavelength range of not less than 1.500 µm but not more than 1.750 µm; the wavelength range of not less than 1.810 µm but not more than 1.880 µm; and the wavelength range of not less than 2.010 µm but not more than 2.380 µm. It is more preferable to perform the measurement based on at least a part of an absorption spectrum that is obtained by irradiating a plant with light that belongs to the wavelength range of not less than 1.500 µm but not more than 1.750 µm or the wavelength range of not less than 2.010 µm but not more than 2.380 µm. It is even more preferable to perform the measurement based on at least a part of an absorption spectrum that is obtained by irradiating a plant with light that belongs to the wavelength range of not less than 1.690 µm but not more than 1.740 µm.

The total amount of fatty acids is measured preferably based on fatty-acid-derived ones of the absorption peaks within the wavelength range (Wavelength Range A) in which the influence of water is negligible. More specifically, it is preferable to perform the measurement based on at least a part of nine absorption peaks that include assigned wavelengths 1.294 µm, 1.712 µm, 1.728 µm, 2.061 µm, 2.175 µm, 2.270 µm, 2.308 µm, 2.342 µm and 2.376 µm, respectively. It is most preferable to perform the measurement based on all these absorption peaks.

How to obtain the absorption spectrum is not particularly limited. In order to unfailingly obtain minute peaks derived from fatty acids such as linolenic acid (described later), it is preferable that the AOTF (AcoustoOptic Tunable Filter) method is employed as a method of scanning with near-infrared light. The measurement is performed at a wavelength interval (measurement slit width) of not more than 2 nm, preferably not less than 0.5 nm but not more than 1.5 nm, and more preferably not less than 0.8 nm but not more than 1.2 nm. For convenience in conducting outdoor observation, it is preferable that a spectrograph for the spectral analysis is of a size and shape that are handy to carry. The distance between a target plant and the spectrograph during the near-infrared spectroscopy is not particularly limited, provided that the spectral analysis can be performed.

A standard curve necessary for the analysis by near-infrared spectroscopy can be prepared in accordance with a known method. Specifically, the analysis by near-infrared spectroscopy may be performed on a standard sample of a fatty acid such as linolenic acid or linoleic acid or of water etc. to obtain a standard curve. One example is as follows. First, a standard sample is analyzed and quantified in advance by gas chromatography. Next, the near-infrared spectroscopy is performed on this sample. Also, a list of peaks corresponding to major fatty acids contained in a plant is prepared, and an absorption peak(s), which is/are preferably unaffected by water, is/are selected from the listed peaks. The absorbance corresponding to the peaks is analyzed by means of multiple regression analysis and is then compared with the data obtained by the gas chromatography analysis. In this way, it is possible to estimate the total amount of fatty acids. Specifically, it is preferable to quantitatively determine the total amount of fatty acids on the basis of all or part of the absorption peaks whose assigned wavelengths are 1.294 µm, 1.712 µm, 1.728 µm, 2.061 µm, 2.175 µm, 2.270 µm, 2.308 µm, 2.342 µm, and 2.376 µm (i.e., fatty-acid-derived absorption peaks unaffected by water). As used herein, the "assigned wavelength" means a value of the approximate median of absorption peak wavelengths. It is preferable that the assigned wavelength allows a margin of error of ±0.001 µm, more preferably ±0.0005 µm.

In a case where linolenic acid which is a fatty acid contained in a plant is analyzed, the analysis can be performed based on at least a part of an absorption spectrum obtained by irradiating a plant with light that belongs to Wavelength Range B. Wavelength Range B is at least one selected from the group consisting of: the wavelength range of not less than 1.350 µm but not more than 1.420 µm; the wavelength range of not less than 1.690 µm but not more than 1.740 µm; the wavelength range of not less than 1.750 µm but not more than 1.785 µm; the wavelength range of not less than 1.905 µm but not more than 1.920 µm; the wavelength range of not less than 1.940 µm but not more than 1.950 µm; the wavelength range of not less than 2.150 µm but not more than 2.180 µm; the wavelength range of not less than 2.190 µm but not more than 2.220 µm; the wavelength range of not less than 2.290 µm but not more than 2.310 µm; the wavelength range of not less than 2.330 µm but not more than 2.350 µm; and the wavelength range of not less than 2.370 µm but not more than 2.400 µm. In order to measure the amount of linolenic acid, it is most preferable to perform the measurement based on both or one of two absorption peaks that are specific to linolenic acid and unaffected by water. The two peaks include the assigned wavelengths 1.712 µm and 2.175 µm, respectively. Note, here, that the "assigned wavelength" means a value of the approximate median of absorption peak wavelengths. It is preferable that the assigned wavelength allows a margin of error of ±0.001 µm, more preferably ±0.0005 µm.

When to perform the measuring step is not particularly limited. In order to obtain the total amount of fatty acids and the amount of linolenic acid which can better reflect the biomass at harvest, it is preferable to perform the measuring step in such a period that overlaps (i) a period of flower bud formation or (ii) a period that is prior to the period of flower bud formation and is related to the formation of flower buds (the period (ii) is hereinafter referred to as a preparatory period before flower bud formation, or a flower-bud-formation preparatory period) of a plant. In order to perform management with higher accuracy, it is more preferable to perform the measuring step in such a period that overlaps the preparatory period before flower bud formation. In particular, in a case where the total amount of fruits (dry weight) or total biomass of above-ground parts of a plant at harvest (dry weight) is used as the plant biomass at harvest, it is preferable to perform the measuring step in such a period that overlaps the period of flower bud formation or the preparatory period before flower bud formation.

In a case where the measuring step is to be performed prior to the period of flower bud formation, a preparatory period before flower bud formation may be identified according to the characteristics of a plant to be measured. For example, in a case where the preparatory period before flower bud formation is roughly known for a target plant, the measuring step may be performed on the basis of this known information. Alternatively, it is possible to easily identify the flower-bud-formation preparatory period by, utilizing the following phenomenon, performing the measuring step and the obtaining step of the present invention in a time-course manner: the percentage of linolenic acid with respect to the total amount of fatty acids shows a characteristic pattern of change when the flower-bud-formation preparatory period starts and when it ends.

The periods of flower bud formation of plants are roughly known in every plant species. Therefore, those skilled in the art can determine this period as needed. The period of flower bud formation can also be easily identified by, in the same manner as in the case of the flower-bud-formation preparatory period, performing the measuring step and the obtaining step of the present invention and examining the percentage of linolenic acid in a time-course manner. Alternatively, the period of flower bud formation may be determined by, for example, checking the appearance of a plant or may be determined from, for example, expression level of a gene responsible for flower bud formation (e.g., LEAFY gene and genes homologous to it). For example, in a case where the plant is pine, the measuring step is performed in the following manner: the period of flower bud formation is determined or estimated by monitoring a change in expression level of genes homologous to LEAFY or by checking the appearance of the tree in the season of flower bud formation. The period of flower bud formation of pine is known to be the year preceding a flowering year. It is estimated, from the percentage of linolenic acid obtained in the measuring step and the obtaining step, that the flower-bud-formation preparatory period is the year preceding the flower bud formation year. This result shows that the present invention can be used to know (estimate) the biomass of pine at the time of harvest that comes in two years.

The measuring step may be performed at a single point (fixed-point measurement), but is more preferably performed at multiple points (multi-point measurement). The multi-point measurement in the case of destructive measurement means performing the measuring step on different parts of an individual plant at the same time or different times. The multi-point measurement in the case of nondestructive measurement means performing the measuring step on a single part or different parts of an individual plant at the same time or different times. In a case where the measuring step is to be performed at different times, each step is preferably performed in such a period that overlaps a period of flower bud formation or a flower-bud-formation preparatory period. The single-point measurement and the multi-point measurement may be performed, for comparison purposes, on a plurality of plants of the same kind (some of them serve as controls) which are grown in substantially the same environment.

### (Obtaining step)

The obtaining step is a step of obtaining the percentage of linolenic acid with respect to the total amount of fatty acids measured in the measuring step. The measured value of the total amount of fatty acids including linolenic acid, and the measured value of a fatty acid (i.e., linolenic acid only), can be obtained by performing the aforementioned measuring step. In the obtaining step, for example, the percentage (e.g., percentage by weight) of linolenic acid with respect to the total amount of fatty acids is obtained by dividing the measured value of the amount of a fatty acid (i.e., linolenic acid only) by the measured value of the total amount of fatty acids including linolenic acid. For example, in a case where the amount of fatty acids is measured by the aforementioned near-infrared spectroscopy, the percentage of linolenic acid with respect to the total amount of fatty acids is obtained by dividing the peak area that reflects the amount of linolenic acid by the peak area that reflects the total amount of fatty acids. It should be noted that the percentage of linolenic acid in a plant with respect to the total amount of fatty acids in the plant may be referred to as a "linolenic acid/total fatty acids" percentage.

### (Estimating step)

The estimating step is a step of estimating the plant biomass at harvest on the basis of the percentage of linolenic acid. The total biomass of any plant at harvest tends to become lower as the "linolenic acid/total fatty acids" percentage becomes higher. In the estimating step, the "linolenic acid/total fatty acids" percentage obtained in the obtaining step is compared with a reference for estimation, and, on the basis of the result of the comparison, the plant biomass at harvest is estimated. It should be noted that the estimation of biomass at harvest found in the estimating step is hereinafter referred to as "estimated biomass at harvest"

A reference used for the estimation (a reference for estimation) can be prepared in advance before the measuring step. For example, a plurality of sets of the "linolenic acid/total fatty acids" percentage and its corresponding biomass at harvest (actual measured value) of a plant (hereinafter referred to as a reference plant) that is the same in kind as a plant whose biomass at harvest is to be estimated are obtained in advance (this step is a reference preparing step). It is preferable that these references include information indicating that different "linolenic acid/total fatty acids" percentages result in corresponding different levels of biomass at harvest. Therefore, the reference plants used here are a plurality of plants of the same kind which are assumed to have different levels of biomass at harvest because of their different growth conditions etc. With the use of these reference plants, the "linolenic acid/total fatty acids" percentages and the corresponding levels of biomass at harvest (actual measured values) of the plurality of plants are obtained at the same time. On the other hand, in a case where the plant whose biomass at harvest is to be estimated is a perennial plant, it is also possible to obtain in advance a plurality of sets of the "linolenic acid/total fatty acids" percentage and its corresponding biomass at harvest (actual measured value) with the use of a single reference plant (which may be the plant whose biomass is to be estimated itself) by obtaining these data at the same time every year (the same season in different years) over several years. The references are preferably plotted on a two-dimensional scatter diagram as multiple plots showing the relationship between the "linolenic acid/total fatty acids" percentage and biomass at harvest, and are more preferably represented as a regression line that shows the relationship between the "linolenic acid/total fatty acids" percentage and biomass at harvest obtained from the multiple plots on the two-dimensional scatter diagram. It should be noted that the estimation can be made more accurately by a regression analysis considering other factors, such as a multiple regression analysis.

Then, the biomass at harvest corresponding to the "linolenic acid/total fatty acids" percentage obtained in the obtaining step is found from the two-dimensional scatter diagram or the regression line etc. The biomass at harvest thus found is used as the estimated biomass at harvest of a plant.

Note that, in the reference preparing step, the "linolenic acid/total fatty acids" percentage can be found in the same manner as in the measuring step and the obtaining step. The "biomass at harvest (actual measured value)" can be obtained by measuring the weight (preferably dry weight) of harvested products after a certain period of time from when the "linolenic acid/total fatty acids" percentage was obtained. The "certain period of time" means a period from when the "linolenic acid/total fatty acids" percentage is obtained to the time of harvest when the obtained "linolenic acid/total fatty acids" percentage has a substantial influence on the biomass at harvest. More specifically, in a case where the plant is an annual plant, the "certain period of time" is a period from when the "linolenic acid/total fatty acids" percentage is obtained to the time of harvest. In a case where the plant is a perennial plant, the "certain period" is determined according to the type of plant. For example, in the case of pine seed, the "certain period" is a period from when the "linolenic acid/total fatty acids" percentage is obtained to the next year or to the time of seed harvest that is in the year after next.

### (Judging step)

The judging step is a step of judging whether or not it is necessary to regulate the plant biomass at harvest, by comparing the estimated biomass at harvest obtained in the estimating step with target biomass at harvest (target value of biomass at harvest).

The target biomass at harvest is set appropriately according to, for example, a production plan in the field of agriculture and forestry. Whether or not it is necessary to regulate the plant biomass at harvest is determined on the basis of whether the difference between the target biomass and the estimated biomass at harvest is within a certain allowable range.

One example of the judging is as follows. 1) It is judged that the biomass does not need any regulation if the estimated biomass at harvest is within ±A% (A is any number set as appropriate) of the target value, 2) it is judged that the biomass needs to be reduced if the estimated biomass at harvest is greater than the target biomass by more than A%, or 3) it is judged that the biomass needs to be increased if the estimated biomass is less than the target biomass by more than A%. A process to regulate the biomass (regulating step) will be described later in detail.

### (One example of step of regulating biomass: Step of changing glutathione concentration in plant)

The method of managing biomass at harvest in accordance with the present invention may include, if needed, after the judging step but before the plant is made use of, a step of, on the basis of the estimated biomass at harvest, regulating the biomass to thereby cause the plant biomass at harvest to be closer to the target value. One example of the regulating step is a step of changing the glutathione concentration in a plant, in particular, changing the daily pattern of glutathione concentration in the plant, while keeping the plant growing even after the estimating step and the judging step until when the plant is made use of.

In order to more effectively regulate the biomass at harvest, the regulating step is preferably performed after the judging step but during or before the period of flower bud formation of the plant. It is more preferable that the regulating step is performed in such a period that overlaps a period before the period of flower bud formation. In the case of performing the regulating step before the period of flower bud formation, it is more preferable to perform the regulating step during a period in which the step can have an impact on flower bud formation (preparatory period before flower bud formation). In particular, in a case where stable production is desired, a process similar to the regulating step may be performed in advance and then the estimating step and the judging step may be performed in a time-course manner. This eventually achieves an improvement in productivity.

There is no particular limitation on how to change the glutathione concentration in a plant. For example, the glutathione concentration may be changed by (i) introducing, into a plant, a polynucleotide that affects the synthesis or catabolism of glutathione or (ii) supplying, to a plant, a biomass regulating agent (a compound other than the above polynucleotide) that affects the level of glutathione in the plant. Supplying a biomass regulating agent to a plant is more preferable.

Specific preferable examples of the polynucleotide that affects the synthesis or catabolism of glutathione include: polynucleotide that codes for gamma-glutamyl cysteine synthetase (this polynucleotide is hereinafter referred to as "GSH1 gene"); and polynucleotide that codes for glutathione-binding plastid-type fructose-1,6-bisphosphate aldolase (this polynucleotide is hereinafter referred to as "FBA gene"). Overexpression of these genes will increase the glutathione concentration in a plant. Other examples of the polynucleotide that affects the synthesis or catabolism of glutathione include various polynucleotides that suppress the expression of the above genes, such as antisense RNA and siRNA.

The GSH1 gene is not limited to a particular kind. Specific examples of the GSH1 gene include: Zinnia elegans (Genbank accession: AB158510); Oryza sativa (Genbank accession: AJ508915); and Nicotiana tabacum (Genbank accession: DQ444219). These genes can also be suitably used in the present invention. The products obtained by translation of these genes also have a chloroplast transit signal peptide in their N-terminal regions as with Arabidopsis thaliana.

Specific examples of the biomass regulating agent that affects the level of glutathione in a plant include: glutathione; glutathione conjugate; active oxygen (e.g., hydrogen peroxide); active nitrogen; polyamine; titanium oxide; jasmonic acid; salicylic acid; cysteine; cystine; heavy metal cadmium; and ferrous ion. These regulating agents are all absorbable by a plant when brought into contact with the plant, and serve to increase the glutathione concentration in the plant. Polyamine can be used to produce hydrogen peroxide. Titanium oxide generates active oxygen upon being exposed to light. Cysteine and cystine are precursors of glutathione. Heavy metal cadmium and ferrous ion are supplied preferably in excess amounts. Of these substances listed above, glutathione is preferable. There are two kinds of glutathione: reduced glutathione (hereinafter referred to as "GSH"); and oxidized glutathione (hereinafter referred to as "GSSG"). GSSG is more preferable because it is physically stable.

When the regulating step is performed whereby the glutathione concentration in a plant which has a daily pattern is reduced toward an optimal level (target level), the biomass at harvest decreases as compared to a case where the regulating step is not performed. When the regulating step is performed whereby the glutathione concentration in a plant which has a daily pattern is increased toward an optimal level (target level), the biomass at harvest increases as compared to a case where the regulating step is not performed. This makes it possible to appropriately manage the plant biomass at harvest. It should be noted that the descriptions in Reference Literature: PCT International Application Publication WO2009/063806 should also be referred to in regard to the step of changing the glutathione concentration in a plant.

### (Another example of biomass regulating step)

The method of managing biomass at harvest in accordance with the present invention may further include, after the judging step, a step of stopping growing the plant, so as not to make use of the plant. Stopping growing the plant in an early stage is advantageous not only in that overproduction is prevented but also in that manpower and cost can be reduced which are otherwise necessary to keep the growth of the plant. Furthermore, by stopping growing the plant and planting a different plant in replacement of the plant, it is possible to optimize the use of a farm land.

### (Step of making use of plant)

According to the method of managing biomass at harvest in accordance with the present invention, plants are managed and grown so that their biomass at harvest will be an appropriate level. The grown plants will eventually be made use of. The meaning of the phrase "making use of a plant" includes (i) harvesting a plant and using it for a certain purpose and (ii) using the plant without harvesting it.

### (Plants to which management method of the present invention is applicable)

The management method of the present invention is applicable to plants of any kind including wild plants and cultivated plants. In view of the management of biomass at harvest, cultivated plants are preferable.

The plants are not limited to a particular kind, and are preferably plants whose above-ground parts are to be made use of. Of these, plants whose fruits or seeds are to be made use of are more preferable. Specific examples of the plants whose above-ground parts are to be made use of include: vegetables such as tomato, green pepper, eggplant, cabbage, crown daisy and green chive; cereal crops and beans such as rice, barley, wheat, rye, green soybean, soybean, red bean and corn; fruits such as orange (citrus fruits), apple, pear, chestnut, grape and peach; trees for use as pulp and timbers such as cedar, sun tree, cypress, eucalyptus, acacia and poplar; ornamental flowers and trees (garden plants) such as lavender, moth orchid and citrus; and other useful plants such as oilseed rape, sunflower, rubber tree, pasture plants, licorice, coral plant, palm and sugar cane.

The plants are preferably perennial (including biennial) grass or woody plants, because their biomass at harvest is more difficult to estimate and manage without the method of the present invention. Of these, perennial woody plants are more preferable. Note, however, that this does not imply any limitation.

It should be noted that the foregoing plants include any plants grown under natural conditions and any plants grown under artificial conditions. Specifically, the meaning of "under artificial conditions" includes, for example, indoor plant cultivation such as cultivation in an greenhouse etc., hydroponic plant cultivation, outdoor plant cultivation such as cultivation in the fields, and plant cultivation in orchards. Specifically, the meaning of "under natural conditions" includes, for example, cultivation in plains, mountains, hills, rivers, lakes and marshes, and oceans.

### (Biomass at harvest)

In the present invention, plant biomass at harvest means biomass (dry weight) of a plant at the time of use thereof. The time of use of the plant means (i) the time of harvest in a case where the plant is made use of by harvesting it and (ii) the time of the use of the plant in a case where the plant is made use of without harvesting it. The biomass at harvest may be total biomass of the plant at harvest; however, in a case where the plant is a plant whose above-ground parts are to be made use of, in particular, in the case of a plant whose fruits or seeds are to be made use of, the biomass at harvest is preferably the total amount of fruits (dry weight) or the total biomass (dry weight) of the above-ground parts of the plant at the time of harvest. It should be noted that, since the degree of richness of a plant body as a whole or above-ground parts of a plant body is relatively correlated with the total amount of fruits on the plant, the total amount of fruits is relatively correlated with the total biomass of the above-ground parts of the plant at harvest.

### (One example of effects brought about by the present invention)

The management method of the present invention makes it possible to manage the productivity of biomass (including seed yield). In particular, when the management method is employed in wide-area plant production management, this will result not only in appropriate production volume and appropriate distribution but also in cost reduction and energy saving which eventually lead to creating a low-carbon society. Furthermore, when the production of agricultural products is managed in a wide area, the method can serve as as a technique to manage the production volume and distribution amount.

The management method of the present invention is more advantageous when employed in management of a larger-scale production system. For example, the management method of the present invention not only improves the growth property of plants but also allows for early knowing of the productivity of the plants (early-stage production estimation). Therefore, the management method is advantageous in that, for example, (i) overproduction in the production system as a whole is prevented and (ii) if it is found that overproduction is expected, a part of the area is used for other plants in an early stage. As such, the management method of the present invention is applicable to an IT (Information Technology) agricultural system which appropriately controls the profitability of a production system.

### [2. System for managing plant biomass at harvest]

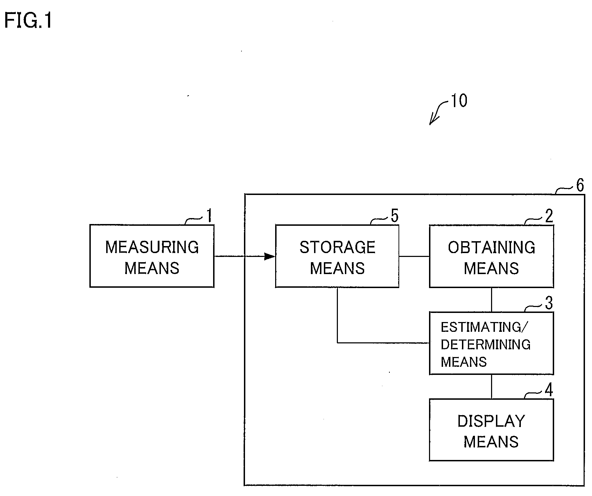

The following description specifically discusses, with reference to Fig. 1, one example of a system that performs the foregoing method of managing plant biomass at harvest.

As illustrated in Fig. 1, a management system 10 at least includes (i) measuring means 1 to measure the amount of fatty acids contained in a plant, (ii) obtaining means 2 to obtain the percentage of the amount of linolenic acid contained in the plant with respect to the total amounts of fatty acids contained in the plant and (iii) estimating/determining means 3. The management system 10 further includes display means 4 and storage means 5. The obtaining means 2, the estimating/determining means 3, the display means 4 and the storage means 5 constitute part of a computer 6. The estimating/determining means 3 serves as (a) estimating means to estimate plant biomass at harvest and (b) determining means to determine, on the basis of the plant biomass at harvest estimated by the estimating means, which process to perform to cause the plant biomass at harvest to be closer to a target value.

The measuring means 1 corresponds to a measuring instrument to perform the measuring step described in the foregoing [1. Method for managing plant biomass at harvest]. Specifically, the measuring means 1 is, for example, gas chromatography equipment, thin-layer chromatography equipment (for destructive measurement), near-infrared spectrometer (for nondestructive measurement), hyperspectrum camera (for nondestructive measurement) or the like. The measuring means 1 supplies, to the storage means 5, measured data concerning the amount of fatty acids contained in a plant.

The storage means 5 stores therein the measured data concerning the amount of fatty acids contained in a plant, which is supplied from the measuring means 1. The storage means 5 further stores therein references for estimation that are for use in the estimating step as described in the foregoing [1. Method for managing plant biomass at harvest].

The obtaining means 2 performs the obtaining step described in the foregoing [1. Method for managing plant biomass at harvest]. More specifically, the obtaining means 2 retrieves, from the storage means 5, measured data concerning the amounts of various fatty acids contained in a plant, and extracts, from the measured data, a measured value of the total amount of fatty acids contained in the plant and a measured value of the amount of linolenic acid contained in the plant. The obtaining means 2 then divides the measured value of the amount of linolenic acid by the measured value of the total amount of fatty acids that include linolenic acid, and thereby obtain the percentage (e.g., percentage by weight) of the amount of linolenic acid contained in the plant with respect to the total amount of fatty acids contained in the plant. The obtained percentage of the amount of linolenic acid with respect to the total amount of fatty acids is supplied from the obtaining means 2 to the estimating/ determining means 3.

The estimating/determining means 3 performs the estimating step and the judging step described in the foregoing [1. Method for managing plant biomass at harvest], and further determines which process to perform in the regulating step. More specifically, the estimating/determining means 3 compares the references for estimation retrieved from the storage means 5 with the percentage of the amount of linolenic acid with respect to the total amount of fatty acids supplied from the obtaining means 2, and, on the basis of the result of the comparison, estimates plant biomass at harvest (execution of the estimating step). The estimating/determining means 3 further judges whether it is necessary to regulate the plant biomass at harvest, by comparing the biomass at harvest estimated in the estimating step with target biomass (target value) at harvest (execution of the judging step). If it is judged that it is necessary to regulate the plant biomass at harvest, the estimating/determining means 3 determines (selects), from various processes for use in the regulating step which processes are stored in the storage means 5, a process that causes the plant biomass at harvest to be closer to the target value (execution of the determining step), and extracts the selected process. The estimating/determining means 3 supplies, to the display means 4, various results such as "biomass at harvest which has been estimated (estimated biomass at harvest)", "result of determination of whether it is necessary to regulate the biomass at harvest", "details of the selected process which is to cause the biomass at harvest to be closer to the target value, details of the result of determination".

The "determining (evaluating) which process to perform to cause the plant biomass at harvest to be closer to the target level" includes, for example, (i) determining whether each process is suitable or not on the basis of the difference between the estimated biomass at harvest and the target value of the biomass at harvest, (ii) extracting the most suitable process from the plurality of processes, and (iii) performing a simulation on how the biomass at harvest will change (improve) if the extracted process is performed.

The determination can be performed more accurately when the storage means 5 stores therein, in advance, various processes that affect the biomass at harvest and their corresponding actual measured changes in the biomass at harvest obtained when the processes are performed on a plant.

The display means 4 is a display device included in the computer 6. Specific examples of the display means 4 include liquid crystal monitors and the like. The display means 4 displays the output from the estimating/determining means 3 in the form that is recognizable to a user of the management system 10.

It should be noted that blocks of the obtaining means 2 and the estimating/determining means 3 which constitute the management system 10 may be realized by a logic circuit (hardware) or may be realized by software as executed by a CPU as described below.

That is, the management system 10 includes a CPU (central processing unit) and memory devices (storage media). The CPU (central processing unit) executes instructions in control programs realizing the functions of the obtaining means 2 and the estimating/determining means 3. The memory devices include a ROM (read only memory) which contains programs, a RAM (random access memory) to which the programs are loaded, and a memory containing the programs and various data. The object of the present invention can also be achieved by mounting to the management system 10 a computer-readable storage medium containing control program code (executable program, intermediate code program, or source program) for the management system 10, which is software realizing the aforementioned functions, in order for the computer (or CPU, MPU) 6 to retrieve and execute the program code contained in the storage medium.

The storage medium may be, for example, tape, such as magnetic tape or cassette tape; a magnetic disk, such as a floppy (Registered Trademark) disk or a hard disk, or an optical disc, such as CD-ROM/MO/MD/DVD/CD-R; a card, such as an IC card (memory card) or an optical card; or a semiconductor memory, such as a mask ROM/EPROM/EEPROM (Registered Trademark)/flash ROM.

The management system 10 may be arranged to be connectable to a communications network so that the program code may be made available over the communications network. The communications network is not limited in any particular manner, and may be, for example, the Internet, an intranet, extranet, LAN, ISDN, VAN, CATV communications network, virtual dedicated network (virtual private network), telephone line network, mobile communications network, or satellite communications network. The transfer medium which makes up the communications network is not limited in any particular manner, and may be, for example, wired line, such as IEEE 1394, USB, electric power line, cable TV line, telephone line, or ADSL line; or wireless, such as infrared radiation (IrDA, remote control), Bluetooth (Registered Trademark), 802.11 wireless, HDR, mobile telephone network, satellite line, or terrestrial digital network. The present invention encompasses a carrier wave or data signal transmission in which the program code is embodied electronically.

In a case where the management system 10 is arranged to be connectable to a communications network, it is also possible to employ a configuration in which measured data outputted from the measuring means 1 is transmitted via the communications network and stored in the storage means 5 which is on a server etc. The obtaining means 2 and the estimating/determining means 3 may also be connected to the storage means 5 (and the measuring means 1) via the communications network. It is also possible to employ a configuration that allows for comprehensive management in which a plurality of measuring means 1 for measuring plants in different places are connected to common storage means 5 via a communications network so that the plant biomass at harvest in the different places can be optimized.
A method (1) in accordance with the present invention is a method for managing plant biomass at harvest, comprising the steps of: a) measuring an amount of fatty acids contained in a plant(s); b) obtaining a percentage of linolenic acid with respect to a total amount of fatty acids that is found from the measurement; and c) estimating the plant biomass at harvest on the basis of the percentage of linolenic acid thus obtained.
A method (2) in accordance with the present invention is the method (1) in which step a) is performed before or during a period of flower bud formation of the plant(s).
A method (3) in accordance with the present invention is the method (1) or (2) in which growth of the plant(s), whose biomass at harvest is managed, is not stopped before, during or after step a).
A method (4) in accordance with the present invention is any one of the methods (1) to (3) in which step a) is performed nondestructively.
A method (5) in accordance with the present invention is any one of the methods (1) to (4) in which step a) includes spectroscopically analyzing near-infrared light contained in light reflected from the plant(s) or near-infrared light contained in light that has passed through the plant(s).
A method (6) in accordance with the present invention is any of the methods (1) to (5) which further includes the step of: step a) includes spectroscopically analyzing near-infrared light contained in light reflected from the plant(s) or near-infrared light contained in light that has passed through the plant(s).
A method (7) in accordance with the present invention is the method (6) in which step d) includes supplying a biomass regulating agent to the plant(s).
A method (8) in accordance with the present invention is the method (7) in which the biomass regulating agent contains glutathione.
A method (9) in accordance with the present invention is any one of the methods (1) to (8) in which the plant biomass at harvest is a total amount of fruits on the plant(s) at harvest or total biomass of above-ground parts of the plant(s) at harvest.

A system (1) in accordance with the present invention is a system for managing plant biomass at harvest, including: measuring means for measuring an amount of fatty acids contained in a plant(s); obtaining means for obtaining a percentage of linolenic acid with respect to a total amount of fatty acids that is found from the measurement; and estimating means for estimating the plant biomass at harvest on the basis of the percentage of linolenic acid thus obtained.

A system (2) in accordance with the present invention is the system (1) which further includes determining means for determining, on the basis of the plant biomass at harvest estimated by the estimating means, which process to perform to cause the plant biomass at harvest to be closer to a target value.

### Examples

The following description discusses the present invention more specifically on the basis of Reference Examples and Examples. Note, however, that the present invention is not limited to these examples.

### [Reference Example 1] Identification of range unaffected by water's absorption spectrum

A preparatory experiment was performed to analyze a near-infrared absorption spectrum of water and a waveform obtained by second-order differentiation of the spectrum. With the use of a near-infrared spectrometer AOTF-NIR Spectrometer Model: C (Infrared Fiber Systems, Inc., USA), light reflected from a measurement spot was measured in the following measurement conditions:
- Wavelength range (Wavelength) 1.300 µm to 2.500 µm
- Wavelength interval (Measurement slit width): 1 nm
- Number of scanning operations (Scan times): 25 times
- Measurement period (Time): 12 seconds

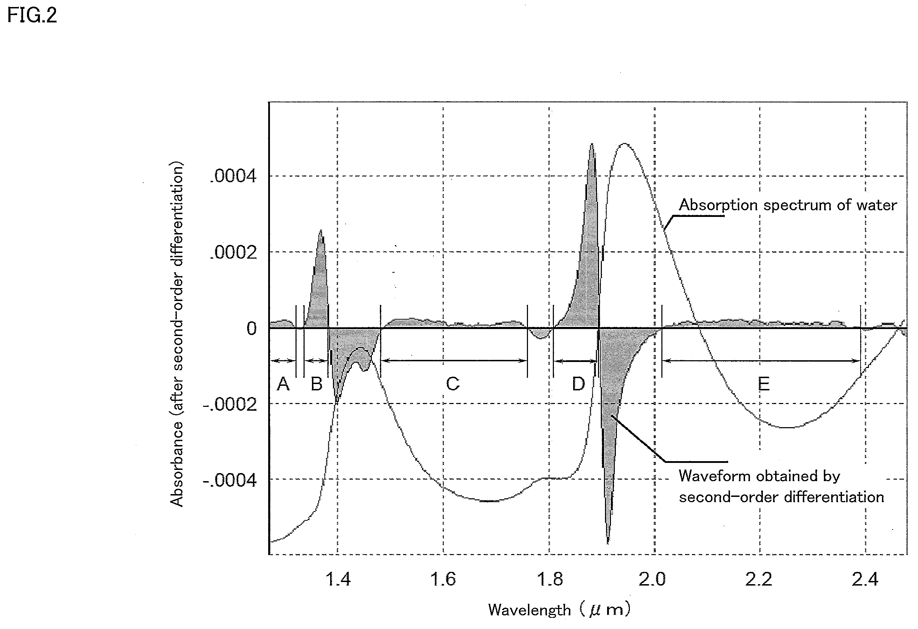

Next, second-order differentiation was performed on the obtained near-infrared absorption spectrum with the use of waveform analysis software 32/AI (Gram). In this way, a graph showing a relationship between wavelength and absorbance was obtained (see Fig. 2). The second-order differentiation was performed by the Savitzky-Golay method using the software with the function set as follows: Derivative 2^{nd}, Degree 2, Points 23.

As shown in Fig. 2, five ranges (a range between Ranges A and B, a range between Ranges B and C, a range between Ranges C and D, a range between Ranges D and E, and a range on the longer wavelength side of Range E) in the wavelength range of not less than 1.250 µm but not more than 2.500 µm overlap the absorption peaks of water. Therefore, Ranges A to E, which are other than the above ranges, were used for the analysis of the amount of fatty acids. These five ranges, i.e., Ranges A to E, are substantially unaffected by absorption by water. Therefore, any or all of Ranges A to E may be used to analyze the amount of fatty acids without the influence of absorption by water.

### [Reference Example 2] Analysis of absorption spectrum of linolenic acid obtained by second-order differentiation

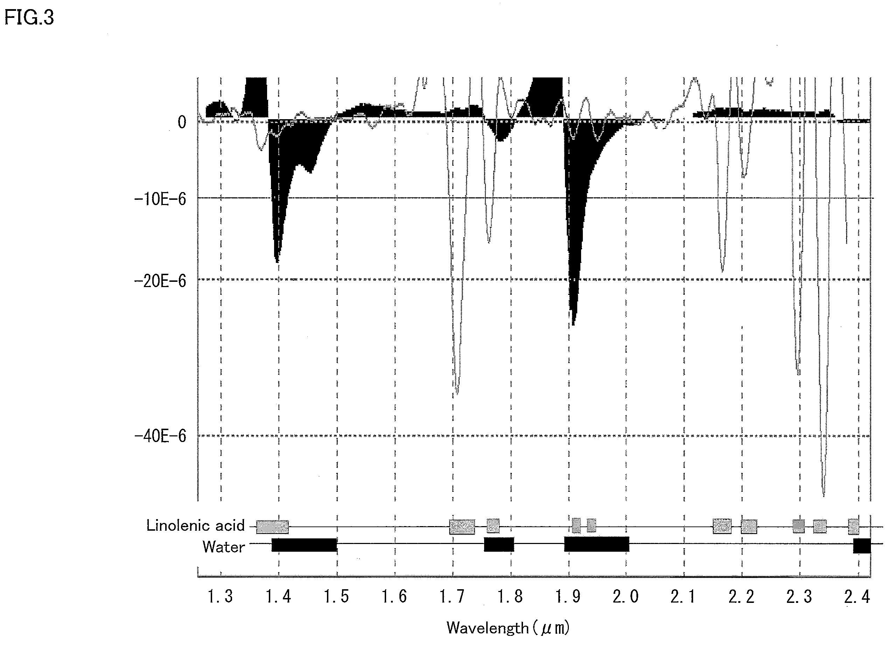

In the same manner as in Reference Example 1, a near-infrared absorption spectrum of a standard sample of linolenic acid (Aldrich USA, Code No. 85,601-0) was obtained. Next, a waveform obtained by second-order differentiation of this absorption spectrum was calculated with the use of waveform analysis software 32/AI (Gram). As shown in Fig. 3, at least ten peaks characteristic of linolenic acid were found within the wavelength range of not less than 1.250 µm but not more than 2.400 µm. Specifically, these peaks were (i) a peak in the wavelength range of not less than 1.350 µm but not more than 1.420 µm, (ii) a peak in the wavelength range of not less than 1.690 µm but not more than 1.740 µm, (iii) a peak in the wavelength range of not less than 1.750 µm but not more than 1.760 µm, (iv) a peak in the wavelength range of not less than 1.910 µm but not more than 1.920 µm, (v) a peak in the wavelength range of not less than 1.940 µm but not more than 1.950 µm, (vi) a peak in the wavelength range of not less than 2.150 µm but not more than 2.180 µm, (vii) a peak in the wavelength range of not less than 2.190 µm but not more than 2.220 µm, (viii) a peak in the wavelength range of not less than 2.290 µm but not more than 2.310 µm, (ix) a peak in the wavelength range of not less than 2.330 µm but not more than 2.350 µm, and (x) a peak in the wavelength range of not less than 2.370 µm but not more than 2.400 µm. Of these peaks, the absorption peaks within the wavelength range unaffected by absorption by water, which are listed in Reference Example 1, are the peak in the wavelength range of not less than 1.690 µm but not more than 1.740 µm, the peak in the wavelength range of not less than 2.150 µm but not more than 2.180 µm, the peak in the wavelength range of not less than 2.190 µm but not more than 2.220 µm, the peak in the wavelength range of not less than 2.290 µm but not more than 2.310 µm, and the peak in the wavelength range of not less than 2.330 µm but not more than 2.350 µm. Note that Fig. 3 also shows a waveform obtained by second-order differentiation of the absorption spectrum of water (filled in with black in Fig. 3) for reference.

### [Reference Example 3] Nondestructive near-infrared spectroscopic measurement of linolenic acid in leaves of orange tree

Using a satsuma orange (Citrus unshiu) tree as a plant sample, the amount of fatty acids contained in the plant sample was nondestructively measured. The satsuma orange tree used here was a tree grown in an orange grove in Arida County, Wakayama Prefecture (in Wakayama Agriculture, Forestry and Fisheries Technology Center). On July 1, 2009, the same process as in Reference Example 1 was performed to measure the amount of fatty acids contained in the satsuma orange tree with the use of AOTF-NIR Spectrometer Model: C (Infrared Fiber Systems, Inc., USA) in the following measurement conditions:
- Wavelength range (Wavelength) 1.25 µm to 2.5 µm
- Wavelength interval (Measurement slit width): 1 nm
- Number of scanning operations (Scan times): 25 times
- Measurement period (Time): 10 seconds

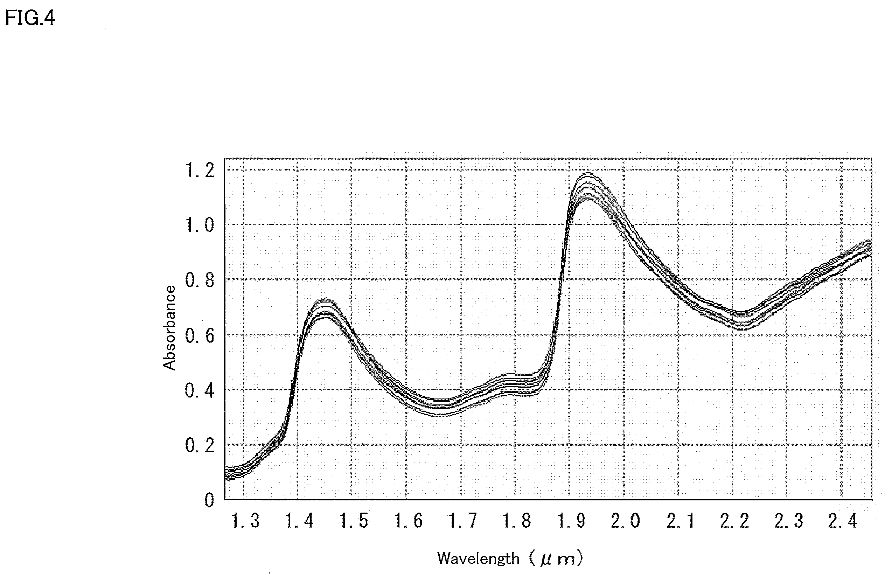

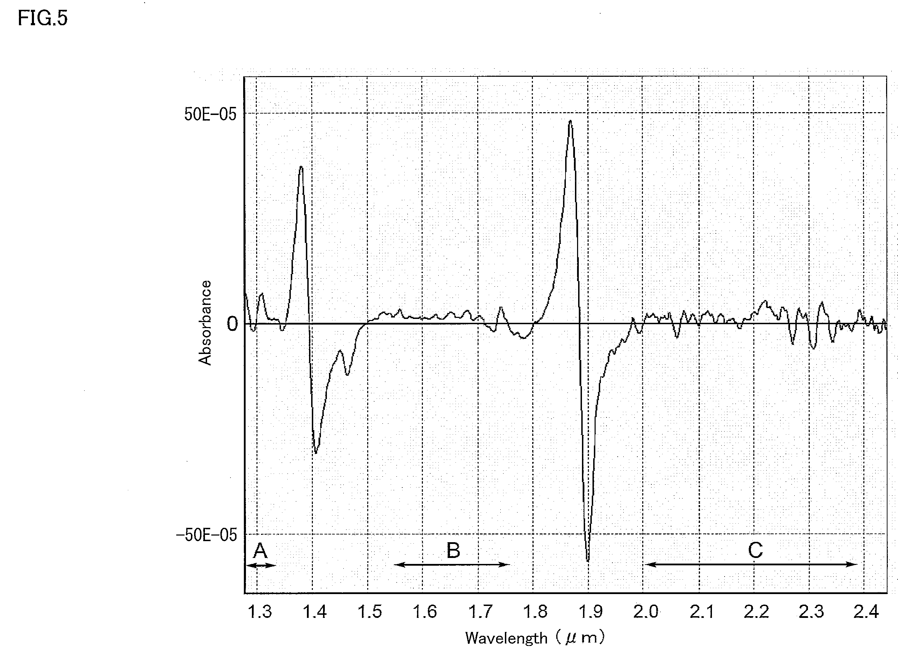

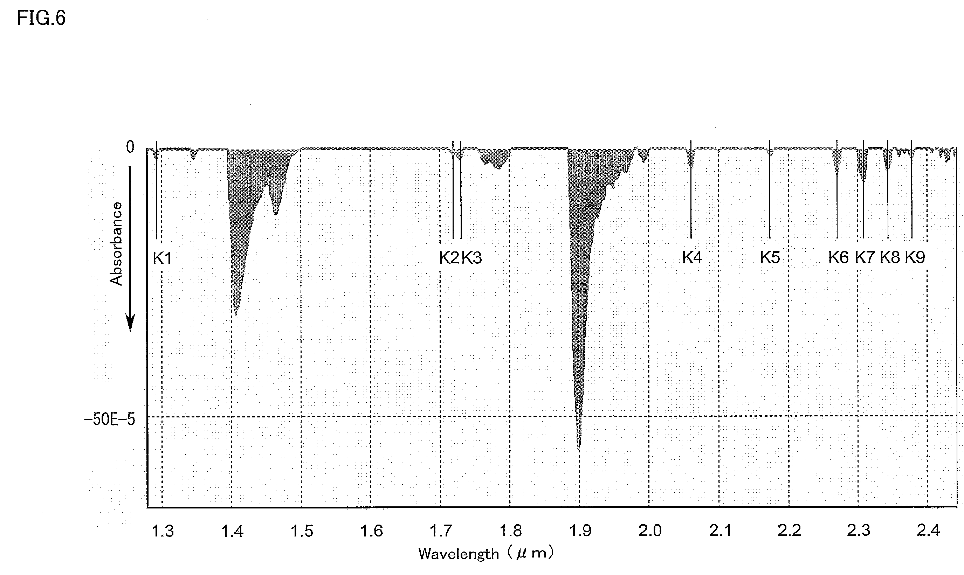

A measurement was performed on light that has passed through a leaf, which was not removed from a branch but was left attached to the branch. The measurement was performed at ten different points on an individual leaf within 3 minutes. Fig. 4 shows ten raw near-infrared absorption spectra. One of these near-infrared absorption spectra was selected, and was differentiated twice (second-order differentiation) with the use of waveform analysis software 32/AI (Gram). In this way, a graph showing a relationship between wavelength and absorbance was obtained (Fig. 5). The second-order differentiation was performed by the Savitzky-Golay method using the software with the function set as follows: Derivative 2^{nd}, Degree 2, Points 27. The wavelength ranges unaffected by water are represented as A, B and C. The ranges where the value indicated by the second derivative of the spectrum is negative are extracted and shown in Fig. 6. The nine peaks indicated as K1 to K9 are those derived from fatty acids. These assigned wavelengths were K1 (1.294 µm), K2 (1.712 µm), K3 (1.728 µm), K4 (2.061 µm), K5 (2.175 µm), K6 (2.270 µm), K7 (2.308 µm), K8 (2.342 µm) and K9 (2.376 µm). The fatty acids corresponding to these nine peaks include plants' major fatty acids (e.g., linolenic acid, linoleic acid, oleic acid, palmitic acid). Of these peaks, K1, K3, K4, K6, K7, K8 and K9 overlap linoleic acid, oleic acid, palmitic acid etc., and therefore cannot be used to measure the amount of linolenic acid. On the other hand, the peaks K2 and K5 are specific to linolenic acid and do not overlap other fatty acids. The fact that the peak K2 or the peak K5 is detectable showed that it is possible to find the amount of linolenic acid even in living leaves of a plant.

A wavelength range to which the absorption spectrum of linolenic acid is assigned was determined by a measurement using a linolenic acid reagent (Aldrich USA, Code No. 85,601-0). Other fatty acids contained in the plant, e.g., linoleic acid, oleic acid and palmitic acid, were also measured in the same manner using reagents (linoleic acid (Aldrich USA, Code No. 85,776-9), oleic acid (Aldrich USA, Code No. 49,043-1) and palmitic acid (Aldrich USA, Code No. 48,961-1)) [these data are not shown]. The results showed that K2 and K5 are the absorption peaks that do not overlap the absorption peaks of the other fatty acids and are specific to linolenic acid.

### [Reference Example 4] Nondestructive measurement on linolenic acid-deficient strain, linolenic acid-excess strain and wild-type strain of Arabidopsis thaliana

The amounts of linolenic acid in the following strains of Arabidopsis thaliana were measured by gas chromatography: (i) a triple mutant (fad3, fad7, fad8/linolenic acid-deficient strain) in which FAD3, FAD7 and FAD8, which are genes responsible for the linolenic acid biosynthetic pathway, are all deleted, (ii) a double mutant (fad7, fad8/linolenic acid-deficient strain) in which FAD7 and FAD8 are deleted, (iii) an FAD3-overexpressing strain (35S-FAD3/linolenic acid-excess strain) and (iv) a wild-type strain (Col wild type). The linolenic acid-deficient strain (triple mutant) and the linolenic acid-excess strain are the same as those disclosed in Patent Literature 1 (Japan Patent No. 4095112 B), and the linolenic acid-deficient strain (double mutant) is the same as a plant disclosed in Reference Literature (Plant Physiology 106, 1609-1614, 1994).

Each strain of Arabidopsis thaliana was grown under the controlled condition where light intensity was approximately 100 µE·m⁻²·S⁻¹, the light and dark cycle was set to 16 hours and 8 hours, and the temperature was 22°C. Four weeks after the start of the cultivation (when flower bud formation is in progress), i.e., on May 23, 2008, an above-ground part (mainly leaf lamina) of each strain was measured.

The amount of linolenic acid was nondestructively analyzed in the same manner as in Reference Example 1. That is, AOTF-NIR Spectrometer Model: C was brought into contact with a leaf of Arabidopsis thaliana to thereby obtain an absorption spectrum, and the absorption spectrum was differentiated twice (second-order differentiation) and analyzed with the use of waveform analysis software 32/AI (Gram) under the following measurement conditions:
- Wavelength range (Wavelength) 1.3 µm to 2.5 µm
- Wavelength interval (Measurement slit width): 1 nm
- Number of scanning operations (Scan times): 25 times on average
- Measurement period (Time): 12 seconds
A waveform was obtained by second-order differentiation of the spectrum with the following configuration: the smoothing coefficient was 79, 11 and the number of points was 17, 19, 23. On the basis of the waveform obtained by second-order differentiation of the spectrum, the amount of linolenic acid and the total amount of fatty acids in the plant were found by the method disclosed in the foregoing Reference Example etc.

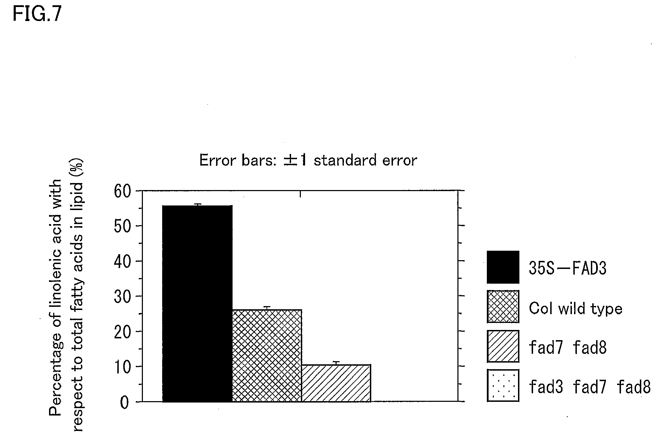

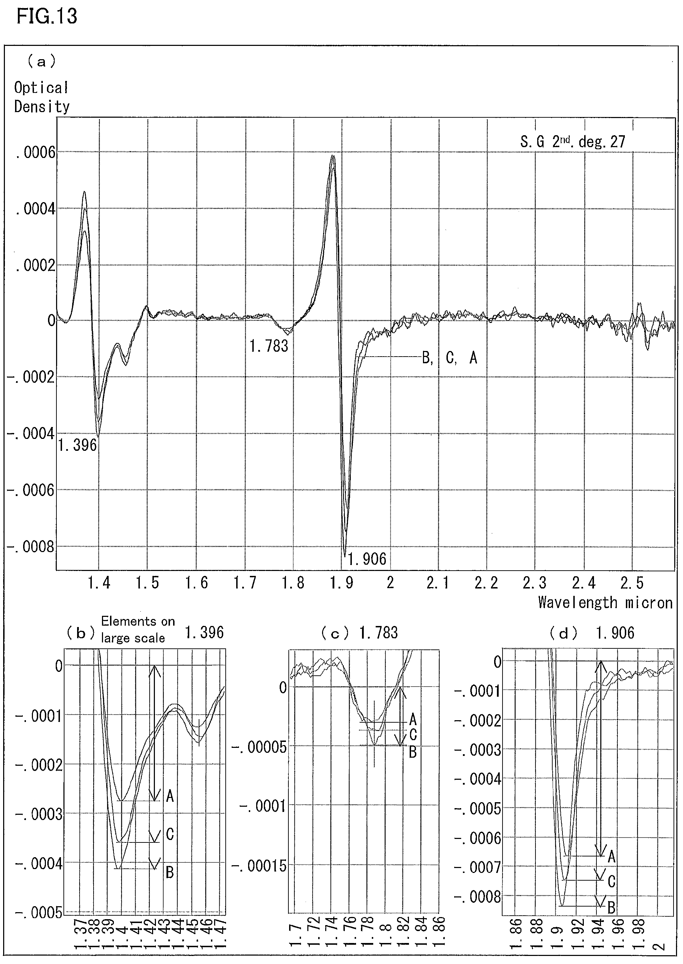

Fig. 7 shows the percentage of linolenic acid with respect to the total amount of fatty acids in each strain of Arabidopsis thaliana, obtained as a result of the gas chromatography analysis. This result showed a similar tendency to the result of measuring the percentage of linolenic acid with respect to the total amount of fatty acids by a nondestructive absorption spectrum analysis (Fig. 13). In Fig. 13, A indicates a waveform obtained by second-order differentiation of the absorption spectrum of the triple mutant, B indicates a waveform obtained by second-order differentiation of the absorption spectrum of the FAD3-overexpressing strain, and C indicates a waveform obtained by second-order differentiation of the absorption spectrum of the wild-type strain. It was found from the waveforms obtained by second-order differentiation of the spectra that the peaks related to linolenic acid are different depending on the amount of linolenic acid. The wavelengths at which the peaks were different depending on the amount of linolenic acid were, for example, 1.396 µm, 1.783 µm, and 1.906 µm.

### [Reference Example 5] Nondestructive measurement of linolenic acid in leaves of coniferous tree

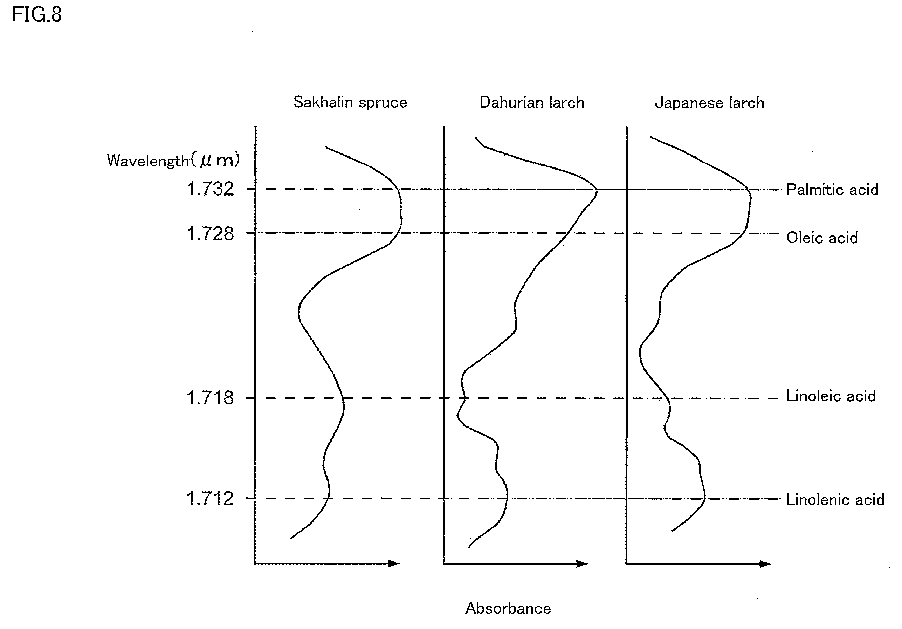

Leaves of the following three different kinds of coniferous trees were analyzed: Sakhalin spruce (Picea glehnii), Dahurian larch (Larix gmelinii) and Japanese larch (Larix kaempferi). These trees are grown in Hokkaido Forestry Research Institute (Bibai city, Hokkaido prefecture). The measurement was performed on June 8, 2008. The amount of linolenic acid was nondestructively analyzed by the same near-infrared spectroscopy as used in Reference Example 1. That is, AOTF-NIR Spectrometer Model: C was brought into contact with the leaves and the reflected light in the near-infrared region of up to 2.600 µm was analyzed. Fig. 8 is a graph showing a relationship between wavelength and absorbance obtained by second-order differentiation of the obtained absorption spectra. Fig. 8 is an enlarged view of the wavelength range 1.690 µm to 1.750 µm. As shown in Fig. 8, each kind of coniferous trees has an absorption peak whose assigned wavelength is 1.712 µm. This result showed that linolenic acid is measurable. The result also showed that other fatty acids such as linoleic acid and oleic acid are also measurable. As such, it was found that the application of the method of the present invention makes it possible to analyze the percentage of linolenic acid with respect to the total amount of fatty acids also for plants other than Arabidopsis thaliana (especially coniferous trees).

### [Example 1] Application of glutathione to linolenic acid-deficient strain, linolenic acid-excess strain and wild-type strain of Arabidopsis thaliana

In Example 1, the biomass of each strain of Arabidopsis thaliana used in Reference Example 4 was measured, and how effective glutathione is to regulate the biomass was tested.

Each strain of Arabidopsis thaliana tested here was grown in the following manner: seeds of three individuals of each strain of Arabidopsis thaliana were sown in a pot, and grown from when the seeds were sown to when the individuals bore seeds (grown for about 2.5 to 3 months) under the controlled condition where light intensity was approximately 100 µE· m⁻²·S⁻¹, the light and dark cycle was set to 16 hours and 8 hours, and the temperature was 22°C. Meanwhile, each kind of glutathione (2 mM GSH aqueous solution or 1 mM GSSG aqueous solution) was applied in an amount of 25 mL to every three Arabidopsis thaliana individuals (except for controls) once a week from the first week to the fifth week (5 times in total).

Above-ground parts of the obtained Arabidopsis thaliana were harvested, and dried for five or more days in a dry house having a humidity of 5% and a temperature of 20°C. After they were thoroughly dried, the total biomass of above-ground parts at harvest (dry weight of the entire above-ground parts) and seed yield (dry weight of harvested seeds) for each pot were measured. As for the linolenic acid-deficient strain (triple mutant), only the total biomass of above-ground parts at harvest was measured.

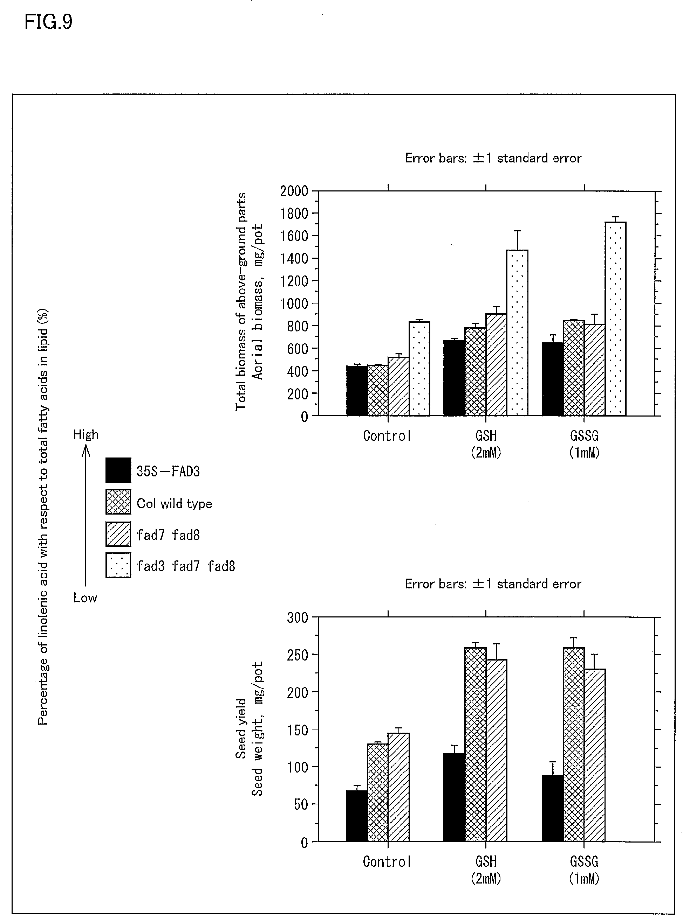

The results are shown in Fig. 9. The percentage (%) of linolenic acid in Fig. 9 corresponds to the result of analyses in Reference Example 4. As is clear from Fig. 9, the results showed that the strains to which glutathione was applied, regardless of the percentage of linolenic acid with respect to the total amount of fatty acids, significantly increased in biomass as compared to the controls. Furthermore, the fact that the higher percentage (%) of linolenic acid results in a smaller above-ground total biomass and smaller seed yield demonstrated that the biomass at harvest can be estimated from the percentage (%) of linolenic acid.

### [Example 2] Relationship between percentage of linolenic acid and number of flowers on orange tree

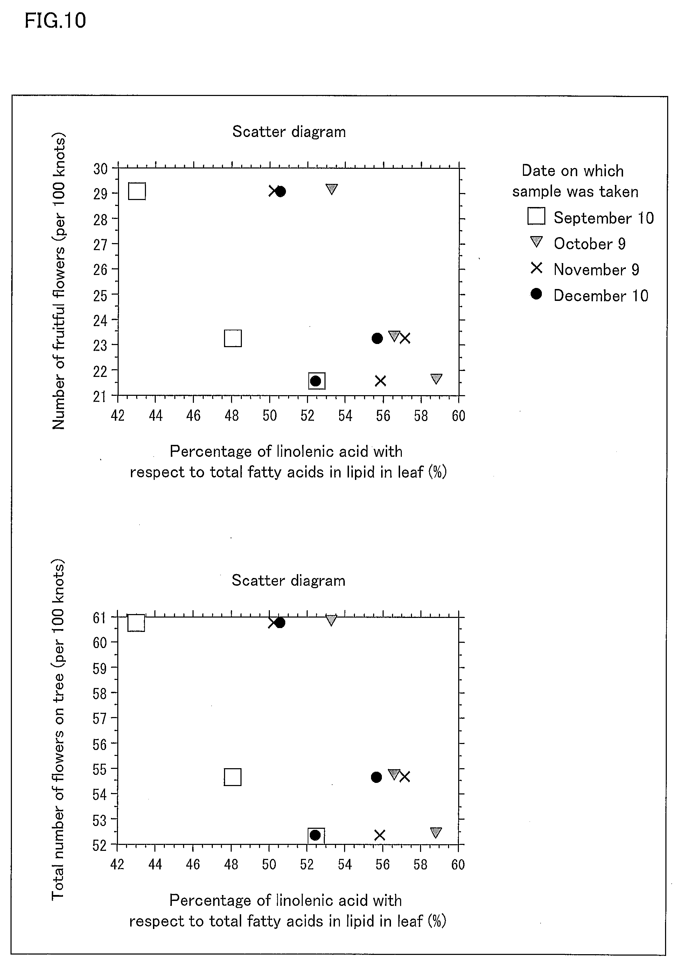

Using three individuals of satsuma orange (Citrus unshiu, cv. Miyagawa wase) as plant samples, the relationship between the percentage of linolenic acid and the number of flowers on trees was examined. The satsuma orange trees used here were grown in an orange grove. Leaves were taken from one-year-old branch on September 10, October 9, November 9 and December 9 in 2009. The leaves were measured for the total amount of fatty acids and the amount of linolenic acid contained therein by gas chromatography, and the percentage of linolenic acid with respect to the total amount of fatty acids was calculated. Furthermore, on May 26, 2010, the number of flowers on these three satsuma orange individuals was counted visually. Fig. 10 shows the relationship between (i) the percentage of linolenic acid with respect to the total amount of fatty acids and (ii) the total number of flowers per 100 knots and the number of fruitful flowers (flowers with leaves) per 100 knots.

As shown in Fig. 10, there was a tendency that the lower percentage of linolenic acid with respect to the total amount of fatty acids results in a larger number of flowers and a larger number of fruitful flowers. The fruitful flowers are known to become good fruits. Therefore, particularly an increase in the number of fruitful flowers means an increase in the biomass at harvest. It should be noted that, as for orange, usually September to October corresponds to an early stage of flower bud formation and the flower bud formation still continues in November and December.

### [Example 3] Relationship between percentage of linolenic acid and amount of cones on pine tree

Using a plurality of Dahurian larch individuals as plant samples, the relationship between the percentage of linolenic acid and the amount of cones on the trees was examined. The Dahurian larch individuals used here were trees grown in Hokkaido Research Organization, Forestry Research Institute, Bibai Center. Leaves were taken from one- to three-year-old (mainly from one-year-old) branches once a month from August to October two years before the year when the amount of cones is to be measured, and the leaves were measured for the total amount of fatty acids and the amount of linolenic acid contained therein by gas chromatography. Then, the percentage of linolenic acid with respect to the total amount of fatty acids was calculated.

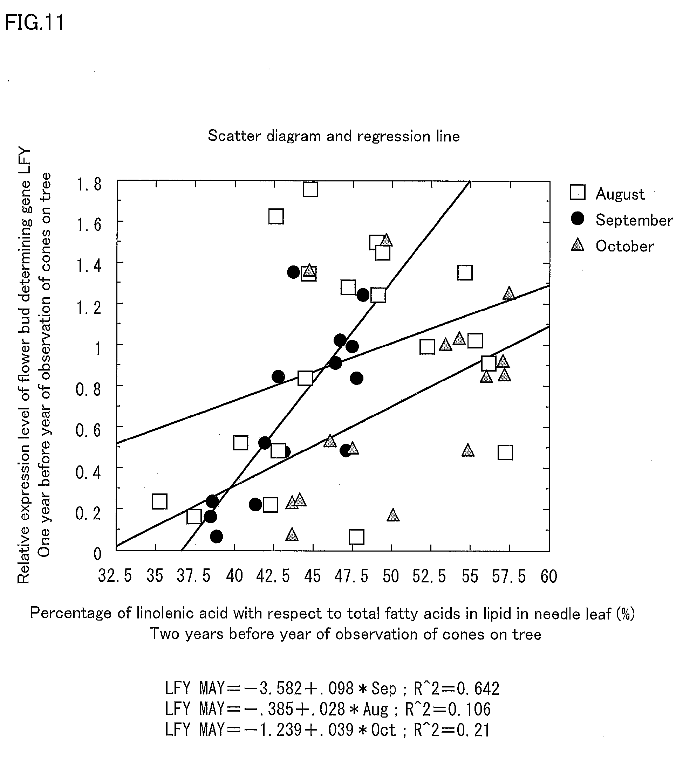

These Dahurian larch individuals were measured for expression levels of the flower bud determining gene LFY in May in the next year. The obtained expression levels were converted into relative levels so that all the measured values were within a range of 0 to 1.8. In the year subsequent to the year when the expression levels of the flower bud determining gene LFY were measured, the amount (number) of cones on the Dahurian larch trees was visually counted. Fig. 11 shows the relationship between (i) the percentage (%) of linolenic acid with respect to total fatty acids measured in August to October and (ii) the expression level of the flower bud determining gene LFY in the year subsequent to the year when the percentage of linolenic acid was measured. Each plot corresponds to one Dahurian larch individual.

As shown in Fig. 11, there was a remarkable tendency that a higher percentage of linolenic acid with respect to total fatty acids results in a relatively higher expression level of the flower bud determining gene LFY in the subsequent year and, in turn, results in an increase of biomass at harvest (the number of cones and total weight of the cones). It should be noted that, although not shown in Fig. 11, the relative expression level of the flower bud determining gene LFY was very closely proportional to the number of cones in the subsequent year, and differences in average weights of the cones between the tested Dahurian larch individuals were substantially negligible statistically.

### [Example 4] Relationship between percentage of linolenic acid and male flowers/female flowers ratio of pine

Using a plurality of Dahurian larch individuals as plant samples, the relationship between the percentage of linolenic acid and the male flowers/female flowers ratio was examined. The Dahurian larch individuals used here were trees grown close to each other. Leaves were taken from the individuals, and the leaves were measured for the total amount of fatty acids and the amount of linolenic acid contained therein by gas chromatography. Then, the percentage of linolenic acid with respect to the total amount of fatty acids was calculated.

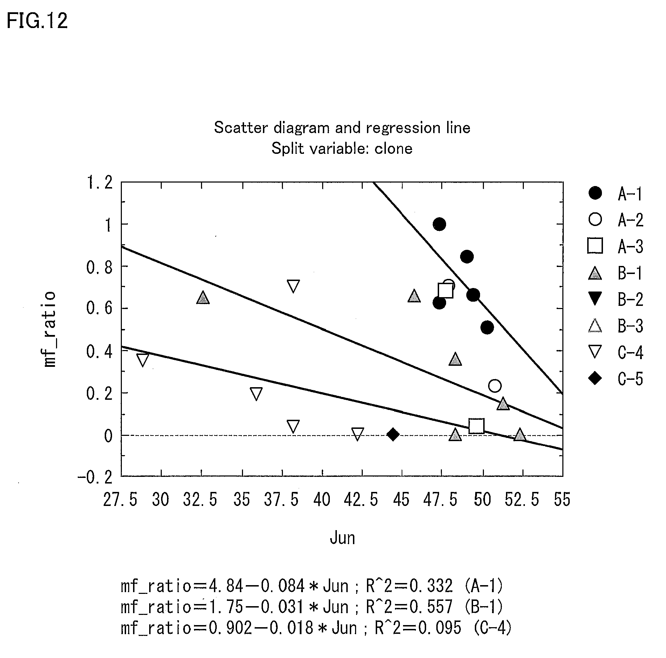

In the year subsequent to the year when the percentage of linolenic acid with respect to the total amount of fatty acids was calculated, the number of open flowers on each individual was visually counted. Then, the male flowers/female flowers ratio (mf_ratio) was found. Fig. 12 shows the relationship between (i) the percentage (%) of linolenic acid with respect to the total amount of fatty acids and (ii) the male flowers/female flowers ratio. Each plot corresponds to one Dahurian larch individual. In Fig. 12, A series (A-1 to A-3: three test groups), B series (B-1 to B-3: three test groups), and C series (C-4 to C-5: two test groups) denote respective different clones. A series is selected as a clone relatively likely to bear cones, and B series is selected as a clone which bears only a very limited number of cones.

The male flowers/female flowers ratio is correlated with the amount of cones and serves as an indicator of the amount of cones. As shown in Fig. 12, it has been shown that the male flowers/female flowers ratio is also correlated closely with the percentage of linolenic acid with respect to the total amount of fatty acids. Note that, when the same measurement was performed a plurality of times and the obtained results were analyzed by means of multiple regression analysis, the correlation coefficient was very high, approximately 0.7 to 0.9 or greater.

### Industrial Applicability

The present invention makes it possible to provide a method and system for knowing in advance plant biomass at harvest and managing the biomass.

### Reference Signs List

- 1: measuring means
- 2: obtaining means
- 3: estimating/ determining means
- 4: display means
- 5: storage means
- 6: computer
- 10: management system

## Claims

1. A method for managing plant biomass at harvest, comprising the steps of:
a) measuring an amount of fatty acids contained in a plant(s); and
b) obtaining a percentage of linolenic acid with respect to a total amount of fatty acids that is found from the measurement;
**characterized by** the further step of:
c) estimating the plant biomass at harvest on the basis of the percentage of linolenic acid thus obtained.

2. The method according to claim 1, wherein step a) is performed before or during a period of flower bud formation of the plant(s).

3. The method according to claim 1 or 2, wherein growth of the plant(s), whose biomass at harvest is managed, is not stopped before, during or after step a).

4. The method according to any one of claims 1 through 3, wherein step a) is performed nondestructively.

5. The method according to any one of claims 1 through 4, wherein step a) includes spectroscopically analyzing near-infrared light contained in light reflected from the plant(s) or near-infrared light contained in light that has passed through the plant(s).

6. A method according to any one of claims 1 through 5, further comprising the step of:
d) regulating the plant biomass by performing, on the basis of the plant biomass at harvest estimated in step c), a process to cause the plant biomass at harvest to be closer to a target value.

7. The method according to claim 6, wherein step d) includes supplying a biomass regulating agent to the plant(s).

8. The method according to claim 7, wherein the biomass regulating agent contains glutathione.

9. The method according to any one of claims 1 through 8, wherein the plant biomass at harvest is a total amount of fruits on the plant(s) at harvest or total biomass of above-ground parts of the plant(s) at harvest.

10. A system for managing plant biomass at harvest, comprising:
measuring means for measuring an amount of fatty acids contained in a plant(s); and
obtaining means for obtaining a percentage of linolenic acid with respect to a total amount of fatty acids that is found from the measurement;
**characterized by**:
estimating means for estimating the plant biomass at harvest on the basis of the percentage of linolenic acid thus obtained.

11. A system according to claim 10, further comprising determining means for determining, on the basis of the plant biomass at harvest estimated by the estimating means, which process to perform to cause the plant biomass at harvest to be closer to a target value.

## Patentansprüche

1. Verfahren zur Bewirtschaftung von Pflanzenbiomasse bei der Ernte, umfassend die Schritte von:
a) Messen einer Menge von Fettsäuren, welche in einer (den) Pflanze(n) enthalten ist, und
b) Bestimmen eines Prozentsatzes von Linolensäure in Bezug auf eine Gesamtmenge von Fettsäuren, welche anhand der Messung gefunden wurde, **gekennzeichnet durch** den weiteren Schritt von:
c) Schätzen der Pflanzenbiomasse bei der Ernte auf Basis des so erhaltenen Prozentsatzes von Linolensäure.

2. Verfahren nach Anspruch 1, wobei Schritt a) vor oder während eines Zeitraums einer Blütenknospenbildung der Pflanze(n) ausgeführt wird.

3. Verfahren nach Anspruch 1 oder 2, wobei das Wachstum der Pflanze(n), deren Biomasse bei der Ernte bewirtschaftet wird, vor, während oder nach Schritt a) nicht angehalten wird.

4. Verfahren nach einem der Ansprüche 1 bis 3, wobei Schritt a) zerstörungsfrei durchgeführt wird.

5. Verfahren nach einem der Ansprüche 1 bis 4, wobei Schritt a) ein spektroskopisches Analysieren von nahem Infrarotlicht einschließt, welches im Licht enthalten ist, welches von der (den) Pflanze(n) reflektiert wird, oder von nahem Infrarotlicht, welches im Licht enthalten ist, welches die Pflanze(n) durchlaufen hat.

6. Verfahren nach einem der Ansprüche 1 bis 5, weiterhin umfassend den Schritt von:
d) Regulieren der Pflanzenbiomasse durch Durchführung eines Verfahrens auf Basis der Pflanzenbiomasse bei der Ernte, welche in Schritt c) geschätzt wurde, um zu bewirken, dass die Pflanzenbiomasse bei der Ernte näher bei einem Zielwert ist.

7. Verfahren nach Anspruch 6, wobei Schritt d) das Aufbringen eines Biomasseregulierenden Mittels auf die Pflanze(n) einschließt.

8. Verfahren nach Anspruch 7, wobei das Biomasse-regulierende Mittel Glutathion enthält.

9. Verfahren nach einem der Ansprüche 1 bis 8, wobei die Pflanzenbiomasse bei der Ernte die Gesamtmenge von Früchten auf der (den) Pflanze(n) bei der Ernte oder die gesamte Biomasse von oberirdischen Teilen der Pflanze(n) bei der Ernte ist.

10. System zum Bewirtschaften von Pflanzenbiomasse bei der Ernte, umfassend:
Messmittel zur Messung einer Menge von Fettsäuren, welche in einer (den) Pflanze(n) enthalten ist,
Gewinnungsmittel zur Gewinnung eines Prozentsatzes von Linolensäure in Bezug auf die Gesamtmenge von Fettsäuren, welche anhand der Messung gefunden wurde, **gekennzeichnet durch**:
Schätzmittel zur Schätzung der Pflanzenbiomasse bei der Ernte auf Basis des so erhaltenden Prozentsatzes von Linolensäure.

11. System nach Anspruch 10, weiterhin umfassend Bestimmungsmittel zur Bestimmung auf Basis der Pflanzenbiomasse bei der Ernte, welche durch das Schätzmittel geschätzt wurde, was für ein Verfahren durchgeführt werden soll, um zu bewirken, dass die Pflanzenbiomasse bei der Ernte näher bei einem Zielwert ist.

## Revendications

1. Procédé de gestion d'une biomasse végétale à la récolte, comprenant les étapes :
a) de mesure de la quantité d'acides gras contenus dans une ou des plantes ;
et
b) d'obtention du pourcentage d'acide linolénique par rapport à la quantité totale d'acides gras déterminée par la mesure ;
**caractérisé par** une étape supplémentaire :
c) d'estimation de la biomasse végétale à la récolte sur la base du pourcentage d'acide linolénique ainsi obtenu.

2. Procédé selon la revendication 1, dans lequel l'étape a) est mise en oeuvre avant ou pendant la période de formation des boutons floraux de la ou des plantes.

3. Procédé selon la revendication 1 ou la revendication 2, dans lequel la croissance de la ou des plantes, dont la biomasse à la récolte est ainsi gérée, n'est stoppée ni avant, ni pendant, ni après l'étape a).

4. Procédé selon l'une quelconque des revendications 1 à 3, dans lequel l'étape a) est mise en oeuvre de façon non destructrice.

5. Procédé selon l'une quelconque des revendications 1 à 4, dans lequel l'étape a) comprend une analyse spectroscopique de la lumière proche infrarouge présente dans la lumière réfléchie par la ou les plantes ou de la lumière proche infrarouge présente dans la lumière ayant traversé la ou les plantes.

6. Procédé selon l'une quelconque des revendications 1 à 5, comprenant, en outre, l'étape :
d) de régulation de la biomasse végétale par mise en oeuvre, sur la base de la biomasse végétale à la récolte estimée à l'étape c), d'un procédé visant à rapprocher la biomasse végétale à la récolte d'une valeur cible.

7. Procédé selon la revendication 6, dans lequel l'étape d) comprend la fourniture d'un agent de régulation de la biomasse à la ou aux plantes.

8. Procédé selon la revendication 7, dans lequel l'agent de régulation de la biomasse contient du glutathion.

9. Procédé selon l'une quelconque des revendications 1 à 8, dans lequel la biomasse végétale à la récolte correspond à la quantité totale de fruits sur la ou les plantes à la récolte ou à la biomasse totale des parties aériennes de la ou des plantes à la récolte.

10. Système de gestion de la biomasse végétale à la récolte, comprenant :
un moyen de mesure pour mesurer la quantité d'acides gras contenus dans la ou les plantes ;
et
un moyen d'obtention pour obtenir le pourcentage d'acide linolénique par rapport à la quantité totale d'acides gras déterminée par la mesure ;
**caractérisé par** la présence :
d'un moyen d'estimation pour estimer la biomasse végétale à la récolte sur la base du pourcentage d'acide linolénique ainsi obtenu.

11. Système selon la revendication 10, comprenant, en outre, un moyen de détermination pour déterminer, sur la base de la biomasse végétale à la récolte estimée par le moyen d'estimation, le procédé à mettre en oeuvre pour rapprocher la biomasse végétale à la récolte d'une valeur cible.
